(19) Europäisches Patentamt
European Patent Office
Office européen des brevets

(11) Publication number: 0 208 523 A2

(12) # EUROPEAN PATENT APPLICATION

(21) Application number: 86305208.0

(22) Date of filing: 04.07.86

(51) Int. Cl.[4]: C 07 K 15/08
A 61 K 37/02, A 01 N 63/02
//C07K5/06, C07K1/14,
A61K35/56

(30) Priority: 08.07.85 GB 8517238
27.11.85 US 802662

(43) Date of publication of application:
14.01.87 Bulletin 87/3

(84) Designated Contracting States:
BE CH DE FR GB IT LI NL SE

(71) Applicant: Usherwood, Peter Norman Russell
35 Normanby Road
Wollaton Nottingham NG8 2TA(GB)

(71) Applicant: Duce, Ian Richard
10 Harles Acres Hickling
Melton Mowbray Leicestershire LE14 3AF(GB)

(71) Applicant: Dell, Anne
24 Grosvenor Road
Hounslow Middlesex TW3 3ER(GB)

(71) Applicant: Taylor, Graham Walter
Norwood North End
Buckhurst Hill Essex IG9 5RA(GB)

(72) Inventor: Usherwood, Peter Norman Russell
35 Normanby Road
Wollaton Nottingham NG8 2TA(GB)

(72) Inventor: Duce, Ian Richard
10 Harles Acres Hickling
Melton Mowbray Leicestershire LE14 3AF(GB)

(72) Inventor: Dell, Anne
24 Grosvenor Road
Hounslow Middlesex TW3 3ER(GB)

(72) Inventor: Taylor, Graham Walter
Norwood North End
Buckhurst Hill Essex IG9 5RA(GB)

(74) Representative: Crespi, Romeo Stefano
Patent Department National Research Development
Corporation 101 Newington Causeway
London SE1 6BU(GB)

(54) **Glutamate antagonists.**

(57) Venoms of New World spiders or the genera genera *Argiope* and *Araneus* e.g. *Argiope trifasciata* and *Araneus gemma* contain active components which have a significantly high level of specific action at glutamate receptors and can be isolated for use as a glutamate antagonist. Moreover, unlike the complex polypeptides referred to above, these compounds are of relatively very low molecular weight (less than 1000) and are accordingly very attractive for insecticidal use and in other applications in which glutamate antagonist activity is desirable.

The isolation purification and characteristics of two such active compounds is described.

EP 0 208 523 A2

Croydon Printing Company Ltd

GLUTAMATE ANTAGONISTS

This invention relates to glutamate receptor antagonists and particularly to components which can be extracted from various venoms or obtained by chemical synthesis.

The venoms produced by members of the arachnid species have in recent years been the target of scientists seeking to identify new chemical entities for use in medicine and agriculture. With the diversity of the species there exists a whole range of venoms with properties ranging from the extremely toxic to the temporary paralysing effect. Unfortunately when prior attempts to identify the structures of interesting components of these venoms have been made it has emerged that the compounds are complex polypeptides which are not appropriate for use in their own right.

It has now been found that venoms of New World spiders of the genera Argiope and Araneus e.g. Argiope trifasciata and Araneus gemma contain active components which have a significantly high level of specific action at glutamate receptors and can be isolated for use as a glutamate antagonist. Moreover, unlike the complex polypeptides referred to above, these compounds are of relatively very low molecular weight (less than 1000) and are accordingly very attractive for insecticidal use and in other applications in which glutamate antagonist activity is desirable.

The present invention comprises compounds derivable from the venom of Argiope trifasciata and of Araneus gemma having the nominal molecular weights 636 and 622 and other physical and chemical characteristics described hereinafter and having glutamate antagonist activity, in a form sufficiently pure to enable such activity to be utilised. These compounds, which we call Argiotoxins, are present in both venoms but in different proportions.

Preferably the glutamate antagonists of the present invention are produced in substantially pure form. Such compounds may be

recovered from the appropriate spider venoms by special purification procedures, these being necessary to separate the desired activity from unwanted and/or harmful components of the venom. The properties of the crude venoms have been referred to in scientific publications in particular by Usherwood, P.N.R. et al, J. Physiol., Paris 1984, 79, 241-245 but no significant purification has hitherto been reported.

The procedure for recovery of the pure glutamate antagonists will be described hereinafter in detail for the venom of *Argiope trifasciata* but is equally applicable to and has been applied to the venom of *Araneus gemma*.

One of the pure compounds from either venom, termed Argiotoxin 636, has an accurate molecular weight (determined by mass measurement) of 636.41. One possible molecular formula consistent with this measurement is $C_{29}H_{52}O_6N_{10}$. Argiotoxin 636 has ultraviolet and nuclear magnetic resonance spectra as described hereinafter. On hydrolysis it gives arginine and aspartic acid which could be accounted for by the following chemical structure:-

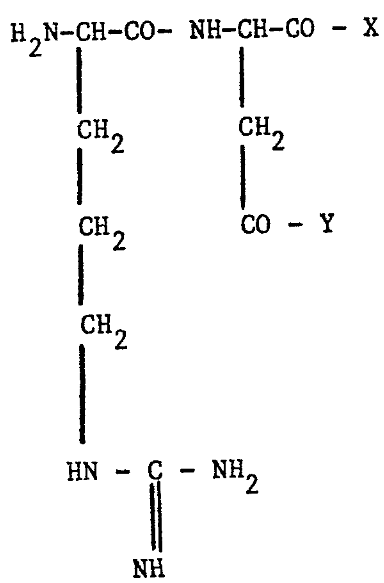

wherein the Groups X and Y, which account for the rest of the molecule contain a 1- substituted 2,4 - dihydroxy benzene group and a primary amido group.

A second pure compound, termed Argiotoxin 622, which appears to be a close homologue of Argiotoxin 636 differing therefrom by one methylene group, has also been isolated. This has an accurate molecular weight of 622.40 which would be consistent with a molecular formula of $C_{28}H_{50}O_6N_{10}$. Argiotoxin 622 also yields arginine and aspartic acid on hydrolysis and is also believed to have the structure postulated for Argiotoxin 636 as referred to above. Argiotoxin 622 has the same ultraviolet spectrum as Argiotoxin 636 and an NMR spectrum which differs only in minor respects from that of Argiotoxin 636. In both Argiotoxins the arginine is N-terminal and is attached to the rest of the molecule through the α carboxy group by a peptide bond.

Isolation, Purification and Partial Characterisation of bioactive factors from Argiope trifasciata and Araneus gemma

(a) Isolation and Purification

100 Venom glands from Argiope trifasciata are homogenised in locust saline, boiled for 4 minutes, centrifuged (11500 g, 10 minutes) and passed through an Amicon YM2 1000 dalton filter. Biological activity is obtained in the low molecular weight filtrate.

The filtrate is dried in vacuo and resuspended in aqueous acetic acid (5% v/v, 250 µl). High performance liquid chromatography (hplc) is carried out on a µBondapak C18 reverse phase column (30 x 0.8 cm), eluting at 2 ml/min isocratically with aqueous acetic acid (5%) for 5 minutes followed by a 15 minute concave gradient (Waters No. 7) to 2.8% propan-2-ol. After injection, major peaks of A280 absorbance elute at 6.2 minutes (Argiotoxin 622) and 8.8 minutes (Argiotoxin 636).

Each peak of activity is further purified on a 5 µm Novapak hplc column eluting at 2 ml/min with acetonitrile:water:trifluoroacetic acid (0:100:0.04, 5 minutes isocratic followed by a 15 minutes

linear gradient to acetonitrile:water:TFA 4:96:0.04 v/v/v); bioactivity elutes with retention times of 18.5 minutes (Argiotoxin 622) and 20.5 minutes (Argiotoxin 636).

This procedure can also be followed using venom of *Araneus gemma* and gives closely similar results within limits of experimental error.

(b) Partial characterisation

1. Argiotoxins possess a characteristic ultraviolet absorbance spectrum. In M/10 HCl $\lambda max = 278$ nm ($\epsilon$ max = 2500) with a shoulder at approximately 284 nm; this is characteristic of an aromatic chromophore. In methanol $\lambda$ max shifts to 280 nm, and in base (M/10 NaOH) the $\lambda$ max shifts bathochromically by 20 nanometers ($\lambda$ max = 298 nm); this is consistent with a phenolic system.

2. The proton NMR spectrum of Argiotoxin 636 isolated as described above has been obtained in deuteromethanol. Two sets of signals were observed in the aromatic region - a doublet at $\delta$ 6.97, 6.94 (J = 8 Hz, 1 proton) and a complex multiplet at $\delta$ 6.33, 6.32, 6.30, 6.27 (2 protons) - corresponding to a 1-substituted -2,4 di-hydroxy aromatic (resorcinol) system. Decoupling experiments indicate that a carbon with at least one proton having a chemical shift of $\delta$3.48 is attached at the 1 position.

Figure 1 shows the spectrum of Argiotoxin 636 in deuteromethanol.

Figure 2 shows the corresponding background spectrum of deuteromethanol alone.

Figure 3 shows the spectrum of Argiotoxin 622 in deuteromethanol.

3. The fast atom bombardment (FAB) mass spectrum of Argiotoxin 636 defines the nominal molecular weight as 636 ($M+H^+$ : m/z 637). Argiotoxins possess an even number of nitrogen atoms. N-acetylation in methanol:acetic anhydride (3:1 v/v, 15 minutes) generates

a di-acetyl derivative (FAB $M+H^+$: 721) whereas acetylation under more forcing conditions in acetic acid:trifluoroacetic anhydride indicates a total of 6 acetylatable groups (OH, NH etc.) in the molecule (FAB $M+H^+$: m/z 889 for the hexa-acetyl species). The presence of a seventh, readily eliminated (presumably hydroxyl) group was inferred from the presence of ions 18 u ( = $H_2O$) below the fully acetylated molecular ion species in the FAB-MS spectrum. Acetylation of arginine results in the addition of 2 acetyl groups on N-acetylation, with a further group added under forcing conditions. The non-peptide part of Argiotoxin 636 thus contains four acetylatable groups none of which is affected by mild N-acetylation.

4. Amino acid analysis indicated the presence of equimolar amounts of arginine and aspartic acid. Edman degradation of Argiotoxin 636 resulted in the formation of PTH-arginine and three substances of molecular weights 480, 615 and 750 (as determined by FAB-MS). The 480 species is the des-arg molecule while the 615 and 750 species are the mono- and di-phenylthiocarbamyl derivatives respectively. The 480 species gave only aspartic acid on amino acid analysis, and shifted to mass 522 (FAB $M+H^+$:m/z 523) on N-acetylation (implying 1 amino group present). Treatment of Argiotoxin 636 with 6M HCl at 105°C for 3 minutes in vacuo converted it into two components giving $M+H^+$ at 620 and 638. Further treatment with $d_4$methanol:methanol (1:1 v:v)/dry HCl shifted the m/z 638 component to 652 and 655 (1:1) but left the 620 component unchanged.

The active materials isolated from spider venoms as hereinbefore described act upon the post-synaptic glutamate receptor/ion channel in arthropod muscle e.g. in the locust Schistocerea gregaria. The compounds of this invention are useful in human medicine, animal husbandry, and in crop protection by application in a suitable carrier. They may be formulated by methods known in

the art to produce anaesthetically effective or central nervous depressant effective pharmaceutical compositions containing a pharmaceutically acceptable carrier or diluent or compositions to terminate or prevent brain damage following stroke. For example as an anaesthetic or central nervous depressant argiotoxin can be applied intraperitoneally through injection at 0.01 - 1 mg/Kg or intravenously at 0.01 - 0.1 mg/Kg. In addition to pharmaceutical uses the compound(s) may be used as research tools in neuroscience in general and in particular for the investigation of neurological disorders such as Huntingdon's chorea, epilepsy, Parkinsonism and senile dementia. Concentrations of active material required for these purposes are in the nanomolar range. The Argiotoxins may also be formulated by known methods to produce pesticidally effective compositions containing an agriculturally acceptable carrier or diluent. As a pesticide the compound(s) can be used for the control of arthropod ectoparasites and applied at concentrations of $10^{-4}$ - $10^{-3}$ per cent. Also as pest control agents in crop protection they may be applied at concentrations of 0.1 - 1 Kg per hectare.

146B

## CLAIMS

1. A compound selected from the group consisting of Argiotoxin 636 and Argiotoxin 622.

2. Argiotoxin 636, being a compound having glutamate receptor antagonist activity, an accurate molecular weight of 636.41, determined by mass measurement, an ultraviolet spectrum in M/10 HCl showing λmax = 278 nm (εmax = 2500) with a shoulder at approximately 284 nm, and in methanol showing λmax = 280 nm, and in M/10 NaOH showing λmax = 298 nm, and an NMR spectrum as shown in Figures 1 and 2 of the accompanying drawings (Figure 1 showing the spectrum in deuteromethanol, Figure 2 showing the background spectrum of deuteromethanol alone).

3. Argiotoxin 622, being a compound having glutamate receptor antagonist activity, an accurate molecular weight of 622.40, determined by mass measurement, ultraviolet spectrum in M/10 HCl showing λmax = 278 nm (εmax = 2500) with a shoulder at approximately 284 nm, and in methanol showing λmax = 280 nm, and in M/10 NaOH showing λmax = 298 nm, and an NMR spectrum as shown in Figures 3 and 2 of the accompanying drawings (Figure 3 showing the spectrum in deuteromethanol, Figure 2 showing the background spectrum of deuteromethanol alone).

4. Argiotoxin 636 according to Claim 1, in substantially pure form.

5. Argiotoxin 622 according to Claim 1, in substantially pure form.

6. A pharmaceutical composition useful for effecting an anaesthetic or central nervous system depressant effect in humans or animals which comprises an anaesthetically effective or central nervous system depressant effect amount of Argiotoxin 636 or Argiotoxin 622 in combination with a pharmaceutically acceptable carrier or diluent.

7. A pharmaceutical composition for terminating or preventing brain damage following stroke which comprises an effective amount of Argiotoxin 636 or 622 in combination with a pharmaceutically acceptable carrier or diluent.

8. A pesticidal composition comprising a pesticidally effective amount of Argiotoxin 636 or Argiotoxin 622 in combination with an agriculturally acceptable carrier or diluent.

9. A method for effecting an anaesthetic or central nervous system depressant effect in humans or animals which comprises administering to a human or animal in need thereof an effective amount of Argiotoxin 636 or Argiotoxin 622 in combination with a pharmaceutical carrier or diluent.

10. A method of terminating or preventing brain damage following stroke in humans or animals which comprises administering, to a human or animal in need thereof an effective amount of Argiotoxin 636 or Argiotoxin 622 in combination with a pharmaceutical carrier or diluent.

11. A method for controlling pests which comprises applying to a locus at which such pests are or will be present a pesticidally effective amount of Argiotoxin 636 or Argiotoxin 622 in combination with a suitable carrier or diluent.

1/3

0208523

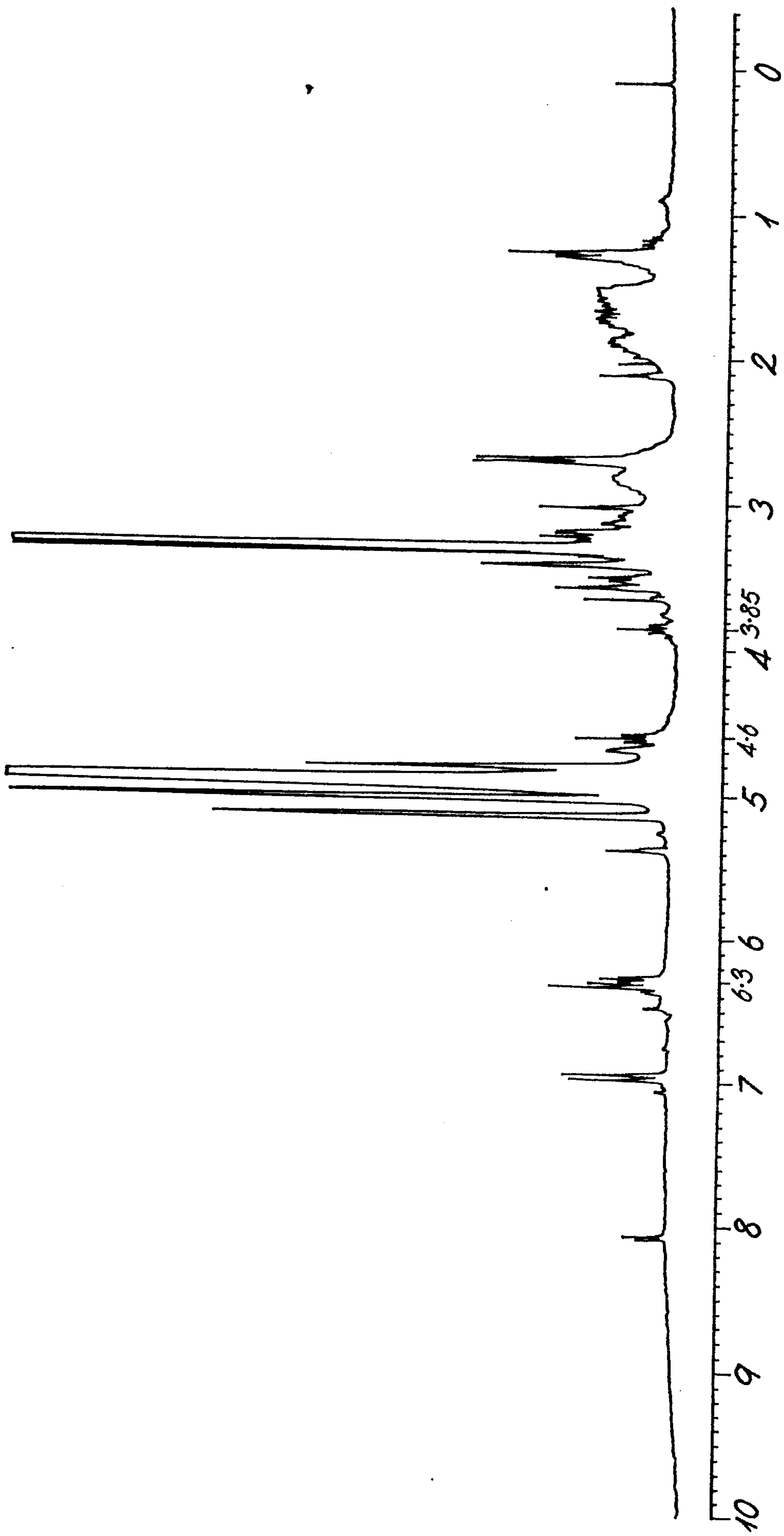


Fig. 1

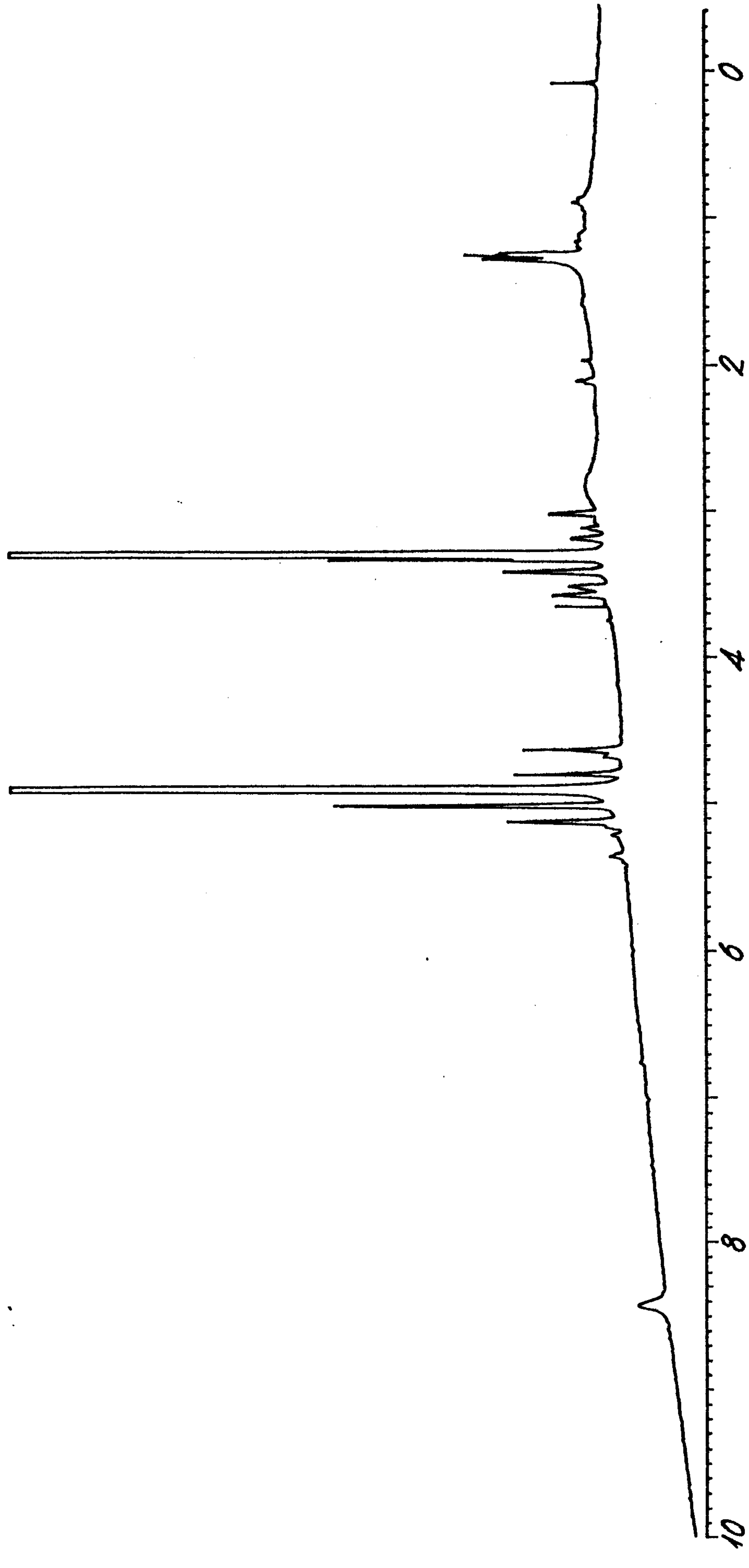


Fig. 2

0208523

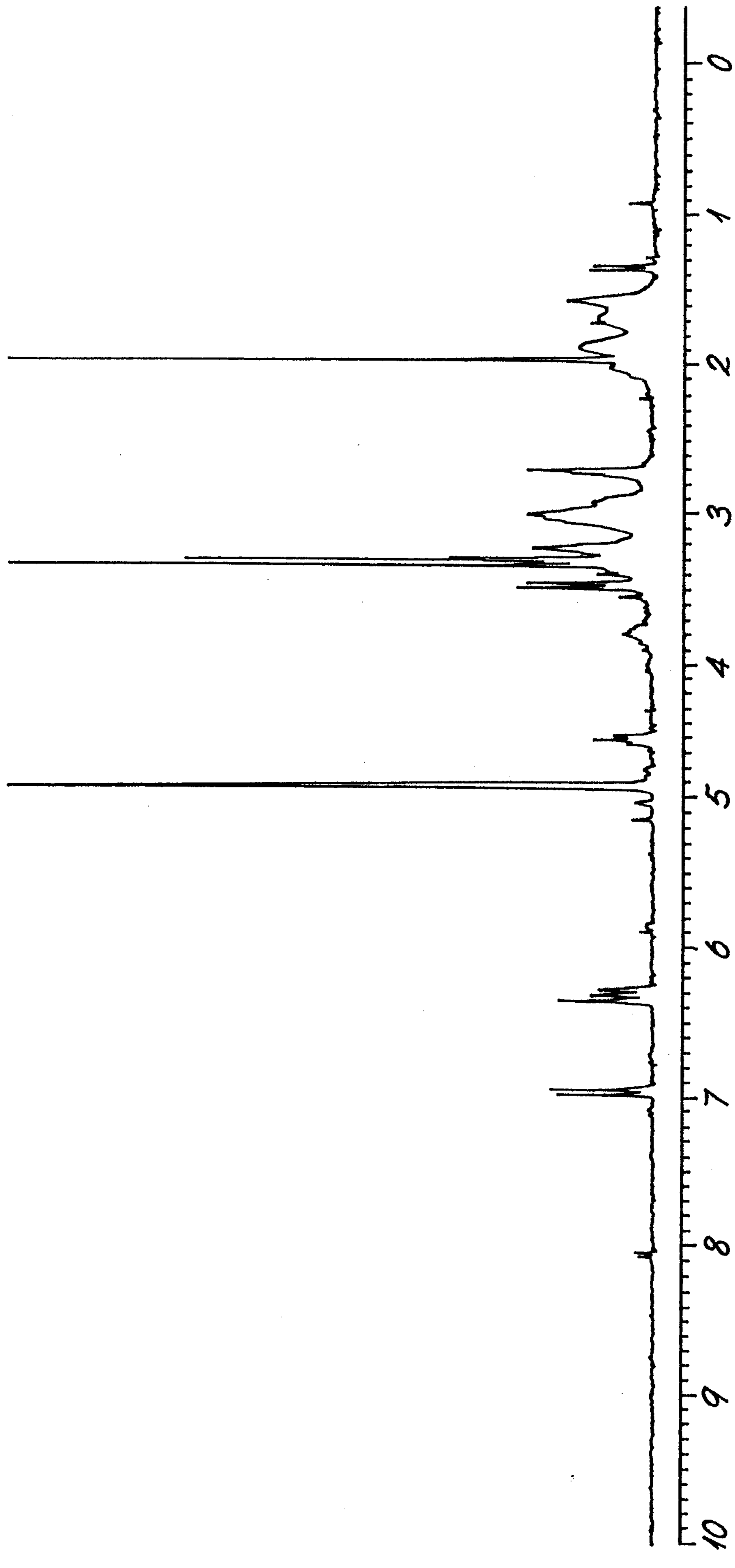


Fig. 3